(19) Europäisches Patentamt
European Patent Office
Office européen des brevets

(11) Publication number: **0 095 944**
**A2**

(12) # EUROPEAN PATENT APPLICATION

(21) Application number: **83303181.8**

(22) Date of filing: **02.06.83**

(51) Int. Cl.³: **A 61 K 31/565**, A 61 K 9/20

(30) Priority: **02.06.82 JP 95070/82**

(43) Date of publication of application: **07.12.83**
**Bulletin 83/49**

(84) Designated Contracting States: **BE CH DE FR GB IT LI NL SE**

(71) Applicant: **Takeda Chemical Industries, Ltd., 27, Doshomachi 2-chome Higashi-ku, Osaka-shi Osaka, 541 (JP)**

(72) Inventor: **Ogawa, Yasuaki,**
**32-503, 7 Nakahozumi 1-chome, Ibaraki Osaka 567 (JP)**
Inventor: **Iga, Katsumi, A-808, 123 Oaza-Saidera, Suita Osaka 565 (JP)**
Inventor: **Satou, Junichi, 19-1,Tohwaen, Nagaokakyo Kyoto 617 (JP)**

(74) Representative: **Woods, Geoffrey Corlett et al, J.A. KEMP & CO. 14 South Square Gray's Inn, London WC1R 5EU (GB)**

(54) **Anti-androgenic preparation for oral cavity administration.**

(57) A pharmaceutical composition, suitable for use in treating prostatomegaly, comprises an anti-androgenically active compound represented by the formula [I]:

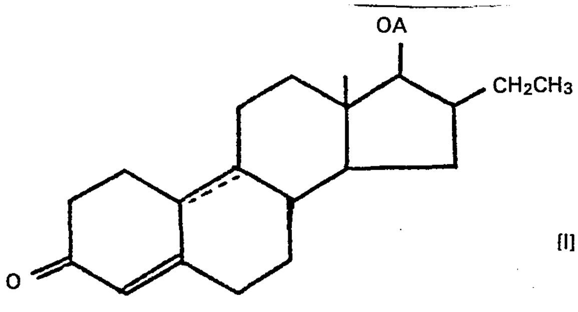

[I]

wherein the bond at the 10-position indicates a carbon-carbon single bond or a carbon-carbon double bond and A indicates a hydrogen atom or an ether or ester residue, and other appropriate components so that the preparation is in the form of an administration unit which is intended to be placed in the oral cavity of a patient to enable the anti-androgenically active compound of formula [I] to be absorbed through the oral mucous membrane. The administration unit has a disintegration time of 30 minutes or longer.

EP 0 095 944 A2

# Anti-Androgenic Preparation for Oral Cavity Administration

## Field of the Invention

This invention relates to a pharmaceutical preparation containing a 16β-ethyl-17β-hydroxy estrane derivative, anti-androgenically active compound, for administration in oral cavity and a method of preparation thereof.

## Description of Prior Arts

16β-ethyl-17β-hydroxy estrane derivatives employed in this invention are represented in the following formula,

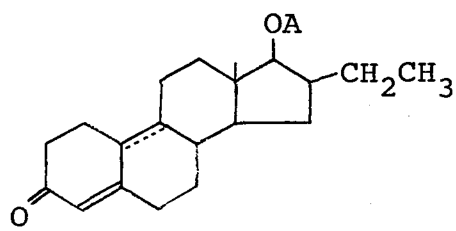

[I]

wherein a dotted line at 10 position indicates a saturated bond or an unsaturated double bond, and A stands for a hydrogen atom, an ether residue, or an ester residue. (US Patent 3856829 and European Patent Application 44495). The compounds belonging to the formula [I] will be often described as estrane [I] in the following. Among them, 16β-ethyl-17β-hydroxy-4-estren-3-one, (code number "TSAA-291") commonly called oxendolone is known as to be effective to remedy prostalomegaly and has been commercially employed in trade name "Prostetin". However, because of its poor solubility in water, Prostetin has been pharmaceutically prepared in the form of an aqueous suspension for injecting into gluteal muscle of a patient. This administration method of injection brings a good absorption in vivo, but entails severe lasting pain around the injection site and needs a treatment by a professional practitioner because the administration method is injection. Consequently, there has been desired the development of other administration, ready to self-medication.

## Summary of the Invention

An object of this invention is to provide a pharmaceutical preparation of estrane [I] for efficient administration other than injection. Other objects of this invention will be apparent in the following descriptions.

In accordance with this invention, these objects are attained by formulating estrane [I] into a preparation so that an administration unit may resist for a period longer than 30 minutes in a disintegration test defined below and by placing said prepared administration unit in oral cavity, in particular, in the upper gingival cavity so that estrane [I] may be absorbed through oral mucous membrane of the site.

## Detailed Description of the Invention

Extensive studies by inventors of this invention, in search of possible administration other than injection, e.g., routine oral route, rectum suppository, through oral cavity membrane, have firstly proved that bioavailability through the oral cavity membrane is greater and superior in terms of scarce fluctuation due to variation in estrane [I], and this administration is easy to self-medication, further is appropriate for industrial preparation with high efficiency.

Secondly, the research on dependency of absorption in vivo upon retention time of a medicament in oral cavity has revealed that absorption is insufficient with a medicament having the retention time of less than 30 minutes. These observations above have led the particular disintegration test as described below to be a factor to define the present invention.

A test sample (a prepared administration unit) is adhesively bonded on an internal surface of an auxiliary tube of small-cylindrical shape made of transparent plastic, and the sample-bonded tube is put on the round bottom of a cylindrical vessel made of transparent plastic with a capacity of 1000 mℓ water. In operation, water in the vessel is warmed and kept at 37 ± 0.5°C. Then an agitator, inverse T-shaped paddle type, is set to agitate the water (100 rpm), keeping some clearance above said sample-loaded tube put on the bottom thereof. A disintegration time is measured at the temperature set as above by visual observation to determine how long a test sample resists in the warm water before its disintegration.

Detail of the apparatus is as follows.

(a) Vessel

Said vessel, made of transparent plastic, is of 1000 mℓ cylinder with round bottom, 160 - 175 mm in height and 50 - 52.5 mm in radius. The upper mouth thereof defines a peripheral flat flange to hold a lid. Said lid, transparent and circular with 140 mm diameter and 4 mm thick, has a groove-like break extending with 12 mm wide from its periphery to the center defining a hole with 16 mm diameter.

(b) Agitator

The journal portion of the shaft has 6.0 - 10.5 mm in diameter and the portion for water submerging has 9.5 - 10.5 mm in diameter and 370 - 400 mm long in total. The paddle, mounted at the shaft bottom edge, is geometrically shaped from a circular plate having 83 mm in diameter and 3.0 - 5.0 mm thick, by two different parallel chords, a chord of 75 ± 1 mm and of 42 ± 1 mm. The intermediate portion between the above two parallel chords defines the dimension of said paddle and is mounted with the 42 mm

chord being down side and through the shaft center.

(c) Auxiliary Tube

A transparent plastic-made tube, 17 mm outer and 12 mm inner diameter and 20 mm long, is tipped with screw thread on both outer ends. Two short tubes with the same diameter dimensions and 2.5 mm long are likewise tipped with screw thread on either inner end to be couplable with the 20 mm tube ends. And two acid-resistant nets, 0.29 mm wire and 0.42 mm opening, are tightly closed with screws at the both end-mouths of the 20 mm tube.

The equipment (a), (b) and (c) above are based on the disintegration test and the dissolution test adopted in the Pharmaceutical Standards in Japan which is disclosed in the Pharmacopeia of Japan, 10th edition.

And it is proved that a distintegration time observed above is comparable with time for a sample medicament unit to remain in oral cavity of an animal or human.

Referring to embodied compounds of estrane [I], A at 17 position may be, in addition to be a hydrogen atom, an acyl radical, e.g., acetyl, propionyl, valeryl, isobutylyl, hexanoyl, heptanoyl, chloroacetyl, enanthyoyl, capryoyl, lauroyl, stearoyl, phenoxyacetyl and benzoyl; or an alkyl, or an ether alkyl, e.g., methyl, ethyl, methoxymethyl, methoxyethyl, ethoxyethyl, propyl, butyl, benzyl, tetrahydropyranyl, tetrahydrofuryl and tetrahydrothienyl; further, acyl or alkyl radicals above may be other straight, branched, saturated or unsaturated ones than specified above.

Referring to specifications for pharmaceutical preparation in this invention, an administration form may be tablet, pill, or suppository, so far as a prepared

administration unit is provided with sufficiently long disintegration time as defined. The preparation is carried out by use of conventional procedures and formulating materials including release controller, vehicle, binder, lubricant, adsorber, and corrigent etc. as known conventionally.

Examples of release controller are cellulose derivatives, e.g. hydroxyethyl-, hydroxypropyl-, hydroxypropylmethyl-, carboxymethyl-cellulose; gelatin, arabic gum, amylopectin starch. A preparation comprising 20 - 60 % of the estrane [I], 7 - 30 % of the release controller and other appropriate formulating materials, tabletted under 1300 - 2000 $Kg/cm^2$ pressure into a size with 6 - 12 mm in diameter and 0.5 - 2.5 mm thick, may provide an adequate disintegration time of from 30 minutes to 10 hours. The requirement for disintegration time of from 30 min. to 10 hrs., preferably from 60 min. to 6 hrs., is attained chiefly by choice of release controller and determination of its formulation ratio. (Percentages (%) above and hereinafter are by weight.)

Examples of vehicle are corn starch, wheat starch, potato starch and other starch; sugars, e.g. sucrose, lactose; inorganic vehicles, e.g. calcium sulfate, calcium phosphate, sodium chloride, precipitated calcium carbonate. As for binder, sugars, starch, dextrin, arabic gum, tragacanth gum, sodium alginate, gelatin, gluten, and other natural binders; methyl-, ethyl-, hydroxyethyl-, hydroxypropyl-cellulose, sodium carboxymethyl cellulose, and other cellulose derivatives; polyvinyl-pyrrolidone, -alcohol, -acetate, macrogol, and other synthetic macromolecular binders are available, and organic macromolecular vehicles or binders are preferable to this invention because they are capable of controlling disintegration of a preparation in aqueous surroundings which is brought about in the test

procedure.

Examples of lubricant are magnesium stearate, talc, synthetic aluminum silicate, ultra fine silicone oxide, higher aliphatic acid, higher alcohol, macrogol, hydrogen-saturated vegetable oil, silicone oil, polyoxyethylene glycol aliphatic acid ester and paraffin. Adsorbents are, for example, ultra fine silicone oxide and active carbon. Corrigents are, for example, natural sweetner; sucrose, glucose, fructose, sorbitol; artificial sweetner; saccharine. Organic acids; for example, citric acid, succinic acid, tartaric acid. Flavor chemical; for example, ℓ-menthol.

Referring further to administration in oral cavity, any site therein, e.g. sublingual cavity, cavity between gingiva and cheek interior, cavity between gingiva and lip interior is allowable. However, conventional troches, sublingual tablets, when put in oral cavity, arouse foreign feel to patients and medicines above are soon chewed or swallowed before they stay for 30 min. or so in the mouth. Actually our experience has concluded that the best is to place an administration unit in the cavity between upper gingiva and upper lip interior. Also it is proved that this best site produces scarce foreign feel and no additional adhesive is necessary to hold and that a preparation placed there may stay so stably that a patient can even take a meal in the meantime.

As for size and shape of administration unit, no restriction is provided with this invention and a sensory test in the form of tablet shows that a tablet having a size from 6 to 12 mm in diameter and from 0.5 to 2.5 mm in thickness will not trouble an adult in taking meal while he holds a tablet in his upper gingival cavity where the tablet will encounter with so less secreted mouth

fluid. Accordingly, sufficient retention time until disappearance is attained on oral mucous membrane. The best preparation experienced in this invention is in the form of tablet with the shape above, which comprises 15 - 20 % of hydroxypropyl- or hydroxypropylmethyl-cellulose, and 0.1 - 10 % of natural sweetner (e.g. glycyrrhizin) and other materials, e.g. disintegration promoter, if necessary.

Up to now, a few reports have disclosed the administration of medicament at the same cavity spot (for instance, Abridgments of reports at 102nd annual convention of the Pharmaceutical Society of Japan, 1982, page 601, 3W, 4-5). But all these disclosures intend the remedy for local treatment on oral cavity area, instead of general treatment like this invention, and pharmaceutical entity disclosed therein is considered to be effective through gastrointestine absorption, instead of through oral membrane. In contrast, the uniqueness of our invention resides in administration in oral cavity for absorption through oral membrane with intention of general treatment.

Embodiments of the Invention

It is to be understood that embodiments of this invention described in the following are for illustration thereof and should not be construed to limit this invention.

Experiment 1

Six different preparations (A through F) containing TSAA-291 were manufactured to exhibit various disintegration times according to the test method as described above. And these preparations were administered to beagle dogs at a dose of 50 mg/dog of TSAA-291, placing an administration unit in their upper gingival cavity and

periodically their blood was collected and blood concentrations thereof were measured to estimate the area under curve (AUC).

Table 1

| Prepared tablet | Disintegration time (min) | Disappearance time at administration site (min) | $AUC_{24}^{0}$ (ng·hr/mℓ) |
|---|---|---|---|
| A | 10 | 10 | 51 |
| B | 30 | 45 | 122 |
| C | 50 | 120 | 396 |
| D | 210 | 318 | 274 |
| E | 420 | 408 | 222 |
| F | 95 | 180 | 297 |

Table 2

| Prepared tablet | Ingredients | | | | | | Dia. of tablet (mm) | Tabletting pressure (Tons/$cm^2$) |
|---|---|---|---|---|---|---|---|---|
| | TSAA-291 | Lactose | Sucrose | CMC | HPC | Mg-stearate | | |
| A | 50 | 100 | | 40 | 2 | 1 | 12 | 0.18 |
| B | 50 | | 100 | 30 | 2 | 1 | 8.5 | 0.5 |
| C | 50 | 100 | | 40 | 16 | 1 | 8.0 | 1.5 |
| D | 50 | 26 | | 40 | 30 | 1 | 6.5 | 1.5 |
| E | 50 | 80 | | | 30 | | 6.5 | 1.5 |
| F | 50 | 15 | Caffeine 25 | | 10 | 1 | 6.5 | 1.5 |

(Notes)

1. Numerals in the column of ingredients are parts by weight, the diameter of tablet is by mm and the tabletting pressure is by 1000 $Kg/cm^2$.
2. CMC is calcium carboxy methyl cellulose.
3. HPC is hydroxy propyl cellulose.

Formulations for the preparations A to F were varied chiefly with respect to the content of the release controller (hydroxy propyl cellulose).

The Table 1 apparently indicates good agreement between disintegration time as measured above and disappearance time as measured at the administration site of beagle dogs. And it also proves that the shorter disintegration time gives the less absorption of TSAA-291 and the preparations capable of lasting for 30 min or more show sufficient absorption values.

Experiment 2

Similar to the foregoing, two preparations (X, Y) having different disintegration times were manufactured and administered to rats in their sublingual cavity at a dose of 10 mg/rat of TSAA-291 and AUC was estimated by periodic blood collections.

Table 3

| Prepared tablet | Disintegration time (min) | Disappearance time at administration site (min) | $AUC_7^0$ (ng·hr/mℓ) |
|---|---|---|---|
| X | 1 | 10 | 89 |
| Y | 60 | 120 | 370 |

Table 4

| Prepared tablet | Ingredients | | | | Dia. of tablet (mm) |
|---|---|---|---|---|---|
| | TSAA-291 | Lactose | CMC | HPC | |
| X | 10 | 10 | 10 | | 2.0 |
| Y | 10 | 20 | | 3 | 2.0 |

The table 3 clearly indicates the same trend as described before.

Example 1

5 g of TSAA-291, 3.5 g of lactose and 1.5 g of hydroxy propyl cellulose were processed in a wet-type granulater. Subsequent to drying, recovered granules were mixed with 0.05 g of magnesium stearate and were tabletted with 1500 $Kg/cm^2$ of tabletting pressure to contain 100 mg of TSAA-291 in one piece. The disintegration time thereof was 120 min.

Example 2

5 g of TSAA-328 (acetate of TSAA-291), 3.5 g of lactose and 1.5 g of hydroxy propyl cellulose were processed in a granulater. Subsequent to drying, granules obtained were mixed with 0.05 g of magnesium stearate and were tabletted with 1500 $Kg/cm^2$ of tabletting pressure to contain 100 mg of TSAA-328 in one piece. The disintegration time thereof was 120 min.

Example 3

1 g of TSAA-330 (caprylate of TSAA-291), 2 g of Witepsol W-35 as suppository base were kneaded under warm condition and fed into a molder to be cooled. The preparation was oily semi-solid suppository for oral cavity, containing 150 mg of TSAA-330 in a unit. The disintegration time thereof was 90 min.

Example 4

5 g of TSAA-291, 3.425 g of lactose, 1.5 g of hydroxy propyl cellulose and 0.075 g of a sweetner (Thaumatin supplied from San-Ei Chemical Industry Ltd.) were wet granulated and dried. Granules obtained were mixed with 0.05 g of magnesium stearate, 0.05 g of ℓ-menthol and tabletted with 1500 $Kg/cm^2$ of tabletting pressure to contain 100 mg of TSAA-291 in one piece. The disintegration time thereof was 120 min.

Example 5

Tablets were prepared by the following formulation with 1500 Kg/$cm^2$ of tabletting pressure.

| Ingredients | parts |
| --- | --- |
| TSAA-291 | 50 |
| Sucrose | 50 |
| Hydroxypropyl cellulose | 40 |
| Glycyrrhizin | 6 |
| Mg-Stearate | 0.5 |
| ℓ-Menthol | 3 |

Tablets formulated above showed about 120 min. in the disintegration test.

## CLAIMS

1. A pharmaceutical preparation comprising an anti-androgenically active compound represented by the formula [I]:

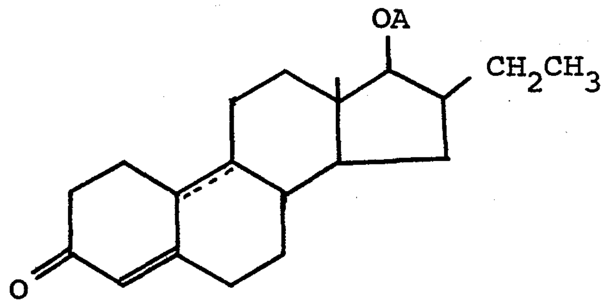

[I]

wherein the bond ═══ at the 10-position indicates a carbon-carbon single bond or a carbon-carbon double bond and A indicates a hydrogen atom or an ether or ester residue, and other appropriate components so that the preparation is in the form of an administration unit which is intended to be placed in the oral cavity of a patient to enable the anti-androgenically active compound of formula [I] to be absorbed through the oral mucous membrane, whereby said administration unit has a disintegration time of 30 minutes or longer as measured by the disintegration test defined below:

Disintegration Test

An administration unit is adhesively bonded to the interior of an auxiliary tube as defined in (c) below and the tube loaded with the administration unit is put into a vessel as defined in (a) below which contains 1000 ml of water at 37 ± 0.5°C. An agitator as defined in (b) below is provided such that its paddle is disposed at the level 5.0 cm above the bottom of said vessel. The agitator is driven at a rate of 100 rpm and time required for disintegration of said administration unit is measured visually at 37 ± 0.5°C.

In the test:

(a) the vessel, made of a transparent plastics, is a 1000 ml cylinder with a round bottom, 160-175 mm in height and 50-52.5 mm in radius; the upper mouth thereof defines a peripheral flat flange to hold a lid and said lid, transparent and circular with 140 mm diameter and 4 mm thick, has a groove-like break extending with 12 mm wide from its periphery to the center defining a hole with 16 mm diameter;

(b) the agitator: the journal portion of the shaft is 6.0-10.5 mm in diameter and the portion for submersion in water 9.5-10.5 mm in diameter and 370-400 mm long in total; the paddle, mounted at the shaft bottom edge, is geometrically shaped from a circular plate having 83 mm in diameter and 3.0-5.0 mm thick, by two different parallel chords, a chord of 75 ± 1 mm and of 42 ± 1 mm; the intermediate portion between the above two parallel chords defines the dimension of said paddle and is mounted with the 42 mm chord being down side and through the shaft center;

(c) the auxiliary tube: a transparent plastics tube, having a 17 mm outer and a 12 mm inner diameter and being 20 mm long, is tipped with screw thread on both outer ends; two short tubes with the same diameters and 2.5 mm long are likewise tipped with screw thread on either inner end to be couplable with the 20 mm tube ends; and two acid-resistant nets, 0.29 mm wire and 0.42 mm opening, are tightly closed with screws at both end-mounts of the 20 mm tube.

2. A preparation according to claim 1, wherein said compound of the formula [I] is 16β-ethyl-17β-hydroxy-4-estren-3-one, 16β-ethyl-17β-acetate-4-estren-3-one, or 16β-ethyl-17β-caprylate-4-estren-3-one.

3. A preparation according to claim 1 or 2, wherein said administration unit is in the form of a pill.

4. A preparation according to claim 1 or 2, wherein, said administration unit is in the form of a tablet.

5. A preparation according to claim 4, wherein the tablet comprises from 20 to 60% of the compound of formula [I] and from 7 to 30% of a release controller, tabletted under from 127486 to 196133 kPa (1300 to 2000 $Kg/cm^2$) pressure.

6. A preparation according to claim 4 or 5, wherein said tablet is from 6 to 12 mm in diameter and from 0.5 to 2.5 mm in thickness.

7. A preparation according to any one of the preceding claims, wherein said oral cavity is the upper gingiva cavity.

8. A preparation according to any one of the preceding claims, wherein said disintegration time is from 30 minutes to 10 hrs.

9. A preparation according to any one of the preceding claims, comprising one or more other components selected from vehicles, release controllers, binders, lubricants, adsorbers and corrigents.

10. A preparation according to claim 9, wherein said release controller is hydroxypropyl cellulose.